(19) Europäisches Patentamt
European Patent Office
Office européen des brevets

(11) Publication number: 0 052 912
B1

(12) **EUROPEAN PATENT SPECIFICATION**

(45) Date of publication of patent specification: **06.02.85**

(21) Application number: **81301640.9**

(22) Date of filing: **14.04.81**

(51) Int. Cl.⁴: **C 07 D 337/14,**
C 07 D 409/04,
C 07 D 409/06,
C 07 D 417/04,
C 07 D 417/06, A 61 K 31/38
// C07C149/43

(54) **7-Fluoro-dibenzo(b,f)thiepin derivatives, their preparation and resolution, and compositions containing them.**

(30) Priority: **24.11.80 US 209434**

(43) Date of publication of application:
**02.06.82 Bulletin 82/22**

(45) Publication of the grant of the patent:
**06.02.85 Bulletin 85/06**

(84) Designated Contracting States:
**AT BE CH DE FR GB IT LI LU NL SE**

(56) References cited:
**EP-A-0 011 067**
**EP-A-0 023 078**
**FR-A-2 312 241**
**GB-A-1 487 280**

**CHEMICAL ABSTRACTS, vol. 86, no. 25, 25th June 1977 abstract 189744k, page 593 COLUMBUS, OHIO (US)**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Rokach, Joshua**
**416 Canterbury Place**
**Chomedey-Laval Quebec (CA)**
Inventor: **Rooney, Clarence S.**
**2902 Hickory Hill Drive**
**Worcester Pennsylvania 19490 (US)**
Inventor: **Cragoe, Edward J.**
**2211 Oak Terrace Drive**
**Lansdale Pennsylvania 19446 (US)**

(74) Representative: **Crampton, Keith John Allen et al**
**D YOUNG & CO 10 Staple Inn**
**London WC1V 7RD (GB)**

(56) References cited:
**CHEMICAL ABSTRACTS, vol. 89, no. 19, 6th November 1978 abstract 163431t, page 563 COLUMBUS, OHIO (US)**

**CHEMICAL ABSTRACTS, vol. 89, no. 13, 25th September 1978 abstract 109152q, page 887 COLUMBUS, OHIO (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 052 912 B1

**Description**

This invention relates to prostaglandin antagonists, which are useful in treating a variety of conditions, such as allergic asthma, where excessive contractile activity of prostaglandins and prostaglandin biosynthetic intermediate compounds occurs.

7- and 8-R-dibenzo[b,f]thiepin derivatives have been described in our European Patent Application No. 11067.

The prostaglandin antagonists of the present invention are 7-fluoro-di-benzo[b,f]thiepins having the structural formula:

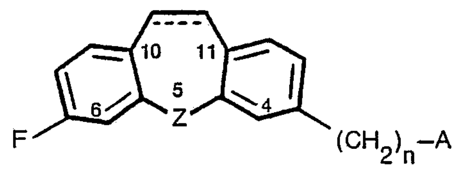

in which n is 0, 1, 2, 3 or 4; Z is thio, sulfinyl, or sulfonyl; A is 5-tetrazolyl, 3-hydroxy-1,2,5-thiadiazol-4-yl, 4-hydroxy-2,5-dioxo-$\Delta^3$-pyrrolin-3-yl, or —CO—$R_2$, where $R_2$ is hydroxy, lower alkyl, lower alkoxy, N,N-diloweralkylaminoloweralkoxy, hydroxyloweralkoxy, carboxyloweralkoxy, amino, N-loweralkylamino, N,N-diloweralkylamino, loweralkylsulfonylamino, carboxyloweralkylamino, carboxamidoloweralkylamino, or 2-imino-3-methylthiazolidine; and the dotted line indicates either an olefinic bond or saturation at the 10,11-position; and the pharmaceutically acceptable salts thereof.

Unless otherwise specifically stated, the terms "loweralkyl" and "loweralkoxy" include straight and branched chain alkyl and alkoxy groups containing 1 to 4 carbon atoms, for example, methyl, ethyl, *n*-propyl, isopropyl, *n*-butyl, methoxy, ethoxy, *n*-propoxy, and isobutoxy. The term "loweralkanoyl" includes straight or branched chain alkanoyl groups having 1 to 4 carbon atoms, for example, formyl, acetyl, propanoyl, butyryl, and isobutyryl.

The present invention also provides a particular class of stereoisomers derived from a racemic mixture of 7-fluorodibenzo[b,f]thiepins having the formula:

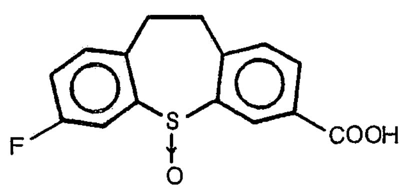

IA

These dibenzo[b,f]thiepin derivatives antagonize the actions of contractile prostaglandins, such as $PGF_{2\alpha}$, $PGG_2$, $PGH_2$, and $TXA_2$. Unexpectedly, the compounds of the present invention are considerably more potent than the corresponding 8-fluoro derivatives in antagonizing the bronchoconstriction caused by $PGF_{2\alpha}$. The use of agents which act as prostaglandin antagonists offers new approaches to therapy in a number of disease states. For example, certain prostaglandins — such as $PGF_{2\alpha}$, $PGG_2$, and $PGH_2$ — are potent contractants of bronchial muscle. Indeed, human asthmatics have been shown to be especially sensitive to the bronchial constricting action of $PGF_{2\alpha}$.

In addition to the involvement of contractile prostaglandins in chronic obstructive lung disease (or asthma), prostaglandins are known to play a role in other allergic conditions, as well as inflammation, diarrhea, hypertension, angina, platelet aggregation, cerebral spasm, premature abortion, and dismenorrhea.

In addition to the prostaglandin antagonist actions, the dibenzo[b,f]thiepins of this invention are antagonists of slow reacting substance of anaphylaxis (SRS—A). This contractile substance is released in the lung tissue in allergic asthma, and antagonism of its actions contributes to alleviation of this disease.

The 7-fluorodibenzo[b,f]thiepins of this invention are prepared according to the following general reaction scheme.

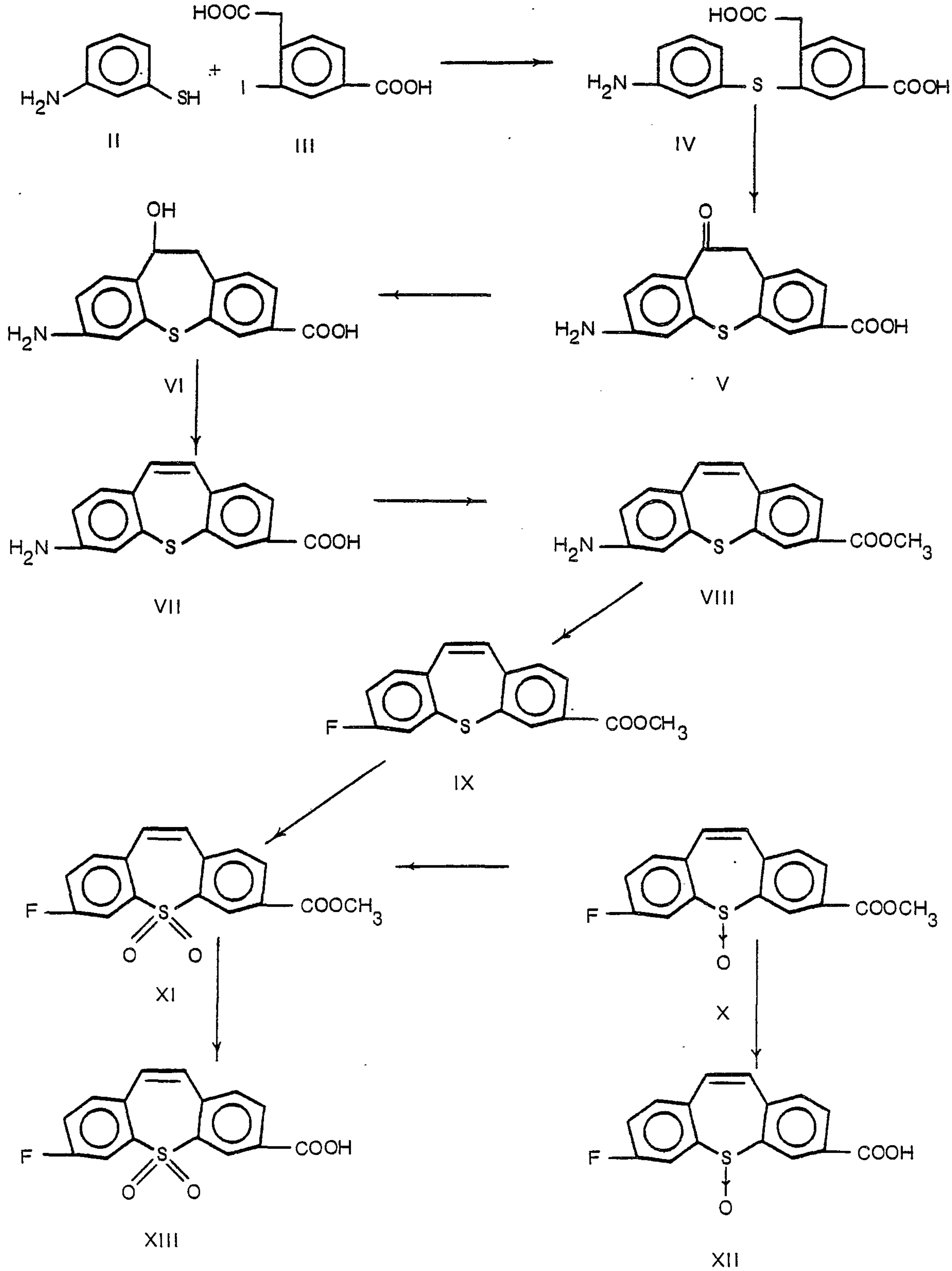

II III IV

VI V

VII VIII

IX

XI X

XIII XII

As a first step in the preparation, 3-aminothiophenol (II) is contacted with 2-iodo-4-carboxyphenylacetic acid (III) in the presence of an alkali metal hydroxide and copper powder to form 2-(3-aminophenylthio)-4-carboxyphenylacetic acid (IV).

Generally, the sulfide-forming reaction is carried out according to the methods described by Jilek et al., Monatsh. Chem. 96, 200 (1965); Protiva et al., Czech. Patent 121,337; C.A. 68 (105, 247t, 1968); and U.S. Patent No. 3,711,489; and by other procedures well known in the art.

The substituted phenylacetic acid IV is then cyclized by heating with a strong acid such as polyphosphoric acid to form 7-amino-10-oxo-10,11-dihydro-dibenzo[b,f]thiepin-3-carboxylic acid (V). Reduction of the oxo substituent of V provides as a first step intermediate the 7-amino-10-hydroxy-3-carboxylic derivative VI which is dehydrated by heating with catalytic amounts of a mineral acid such as sulfuric acid or p-toluenesulfonic acid to form the corresponding 7-amino-dibenzo[b,f]thiepin-3-carboxylic acid VII, and esterified to the lower alkyl ester VIII. This amino substituted ester is then reacted with sodium nitrite in fluoboric acid to form the diazonium fluoborate salt which is converted by pyrolysis to the 7-fluoro-dibenzo[b,f]thiepin-3-carboalkoxy compound IX.

Compound IX can, in a controlled oxidation with peroxides such as hydrogen peroxide or organic peroxy acids such as m-chloroperbenzoic acid, yield compound X. For example, compound IX may be oxidized with one equivalent of organic peroxides such as m-chloroperbenzoic acid, or with hydrogen peroxide in hydroxylic solvents such as alcohols, or organic acids such as acetic acid, at temperatures below 30°C., to yield X. Compounds IX and X may also be oxidized with excess organic peroxides such as m-chloroperbenzoic acid at room temperature, or with peroxides such as hydrogen peroxide in acidic medium such as acetic acid at temperatures between 80° and 100°C. to yield XI.

Compounds X and XI are then hydrolyzed using an aqueous solution of a strong base to produce the respective acids XII and XIII.

In an alterantive method of preparation of the compounds of the present invention, the diazotization step is eliminated by use of an alternative starting material in accordance with the following reaction scheme.

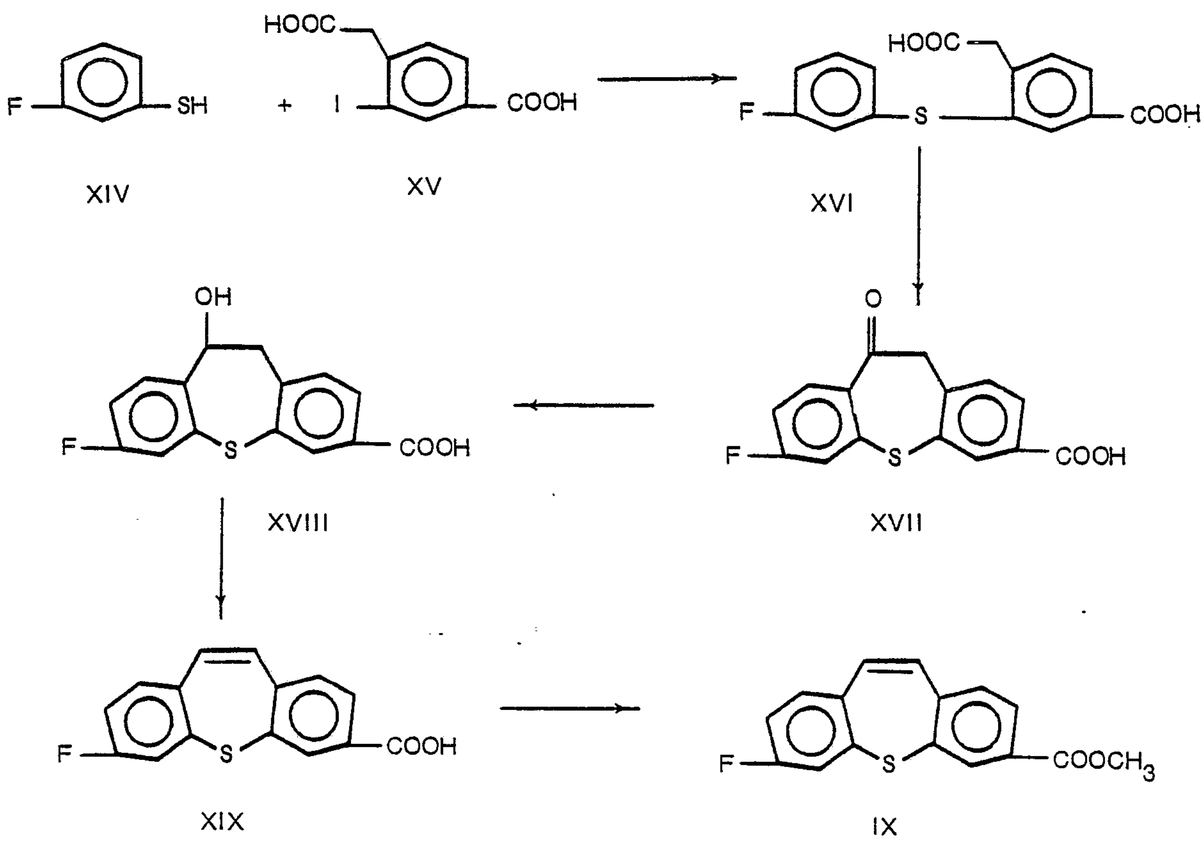

XIV

XV

XVI

XVIII

XVII

XIX

IX

Reaction of 3-fluoro-thiophenol (XIV) and 2-iodo-4-carboxyphenylacetic acid (XV) by heating in the presence of copper powder and an aqueous solution of a strong base forms 2-(3-fluorophenylthio)-4-carboxyphenylacetic acid (XVI). This substituted phenylacetic acid is then cyclized by heating in the presence of a strong mineral acid such as polyphosphoric acid to produce the 10-oxo-10,11-dihydro-dibenzothiepin (XVII) which is reduced with sodium borohydride to produce the corresponding 10-hydroxy compound XVIII. This 10-hydroxy compound is then dehydrated by heating with a catalytic amount of a strong mineral acid such as sulfuric or p-toluenesulfonic to form the 7-fluorodibenzo[b,f]thiepin-3-carboxylic acid (XIX) which is esterified to form intermediate IX shown hereinabove.

The corresponding dibenzothiepins having a tetrazole substituent at the 3-position are prepared from the corresponding 3-cyano compounds by methods known in the art as applied to this series of compounds. Thus, the compounds of the present invention having a tetrazolyl substituent are prepared by the following reaction scheme.

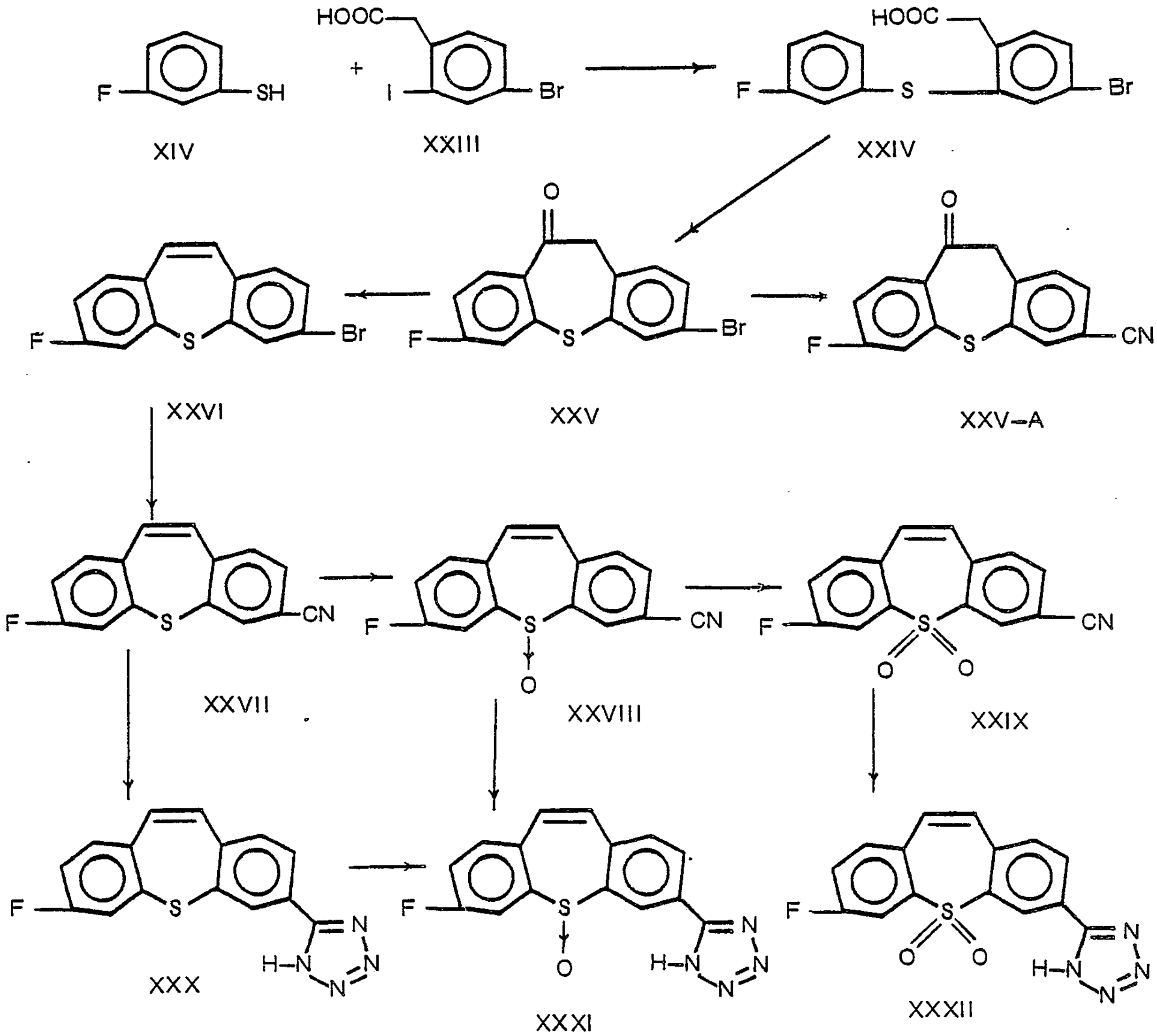

XIV XXIII XXIV

XXVI XXV XXV-A

XXVII XXVIII XXIX

XXX XXXI XXXII

The carboxylic acid XXIV is transformed into the 3-bromo-7-fluoro-11-oxo-10,11-dihydrodibenzo[b,f]thiepin by first conversion to the acid halide with thionyl or phosphoryl halide followed by Friedel-Crafts cyclization with a Lewis acid such as aluminum chloride to give XXV. Reduction of the ketone XXV with alkali metal borohydride, followed by heating with catalytic amounts of a mineral acid, such as sulfuric acid or toluenesulfonic acid provides the 3-bromo-7-fluoro-dibenzo[b,f]thiepin (XXVI).

The 3-bromo derivative XXVI is then converted to the 3-nitrile XXVII by reaction with cuprous cyanide in a high boiling polar solvent such as dimethylformamide, N-methylpyrrolidone, and the like.

The 3-cyano derivative XXVII may be hydrolyzed with aqueous mineral acid or base to give the dibenzo[b,f]thiepin-3-carboxylic acid XIX. The 3-cyano compound XXVII may also be reacted with azide ion at reflux in an inert solvent such as dimethylformamide, hexamethylphosphorictriamide and the like for 1/4 to 18 hours to give the tetrazole derivative XXX. Alternatively, the cyano intermediate XXVII may be oxidized with organic peroxides such as peroxy acids — for example, m-chloroperbenzoic acid and the like — in a stepwise fashion to the corresponding sulfoxide XXVIII and sulfone XXIX, controlling the molar ratio of oxidant to reductant. This determines the oxidation level of the sulfur. For example, a 1:1 molar ratio results largely in the production of sulfoxide XXVIII. In contrast, a 2 to 3 molar excess of oxidant results in a yield predominantly comprising the sulfone XXIX.

Reaction of XXVIII and XXIX with azide ion as described above provides the tetrazoles XXXI and XXXII, respectively.

Tetrazole XXX may be oxidized with peroxides such as hydrogen peroxide in acidic medium, such as acetic acid, to yield compound XXXI.

In addition to their therapeutic properties, as noted above, the 3-carboxylic acid derivatives of this invention serve as valuable intermediates in the preparation of other variously substituted thiepins of formula I. Thus for example, the 3-carboxylic acid of formula XIX may be converted readily into the corresponding acid halide, preferably the acid chloride, by treating the carboxylic acid with a thionyl halide,

preferably thionyl chloride. The resulting 3-halocarbonyl-7-fluoro-dibenzo[b,f]thiepin, i.e., the 3-chlorocarbonyl compound of formula XXXIII, then may be treated with various well known reagents to form desired ester and amide derivatives. These reactions are illustrated in the following reaction scheme, it being understood that they are equally applicable to the 3-carboxylic acids of formulas XII and XIII.

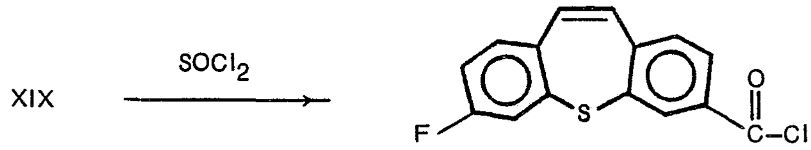


XXXIII

Thus, for example, the chlorocarbonyl compound of formula XXXIII may be treated:

(a) with a loweralkanol such as, for example, methanol, ethanol, 2-propanol, butanol and 2-butanol, to form the corresponding loweralkyl ester XXXIV:

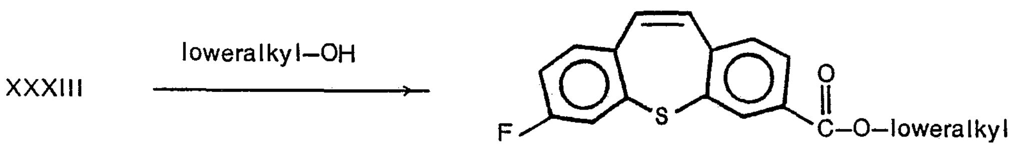


XXXIV

(b) with ammonia to form the corresponding carboxamide XXXV:

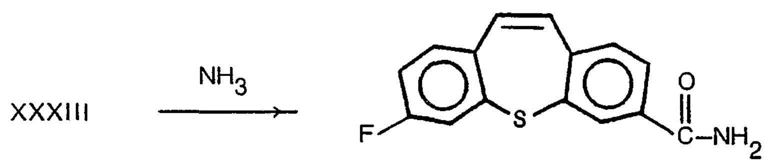


XXXV

(c) with an N-loweralkylamine such as, for example, methylamine, ethylamine, propylamine, isopropylamine, and butylamine, or an N,N-diloweralkylamine such as, for example, dimethylamine, diethylamine, dipropylamine, and dibutylamine, to form the corresponding N-loweralkylcarboxamide XXXVI or N,N-diloweralkylcarboxamide XXXVII:

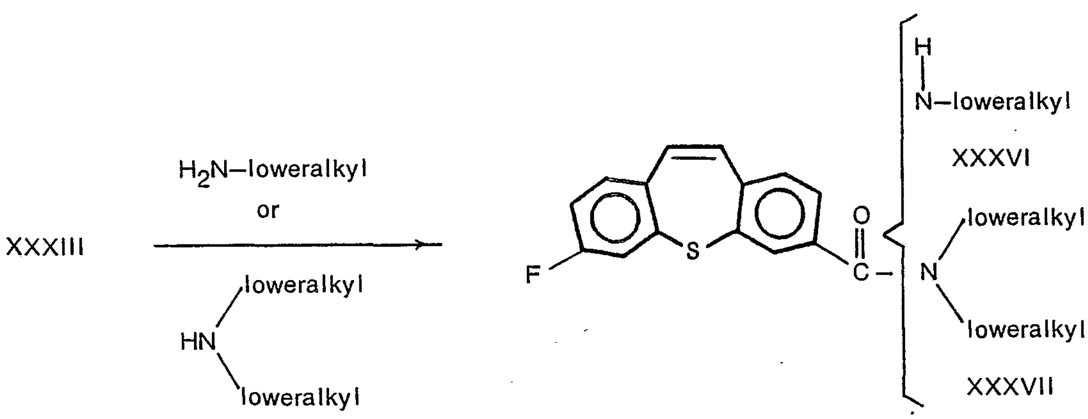

(d) with a loweralkylsulphonamide such as, for example, methanesulphonamide, ethanesulphonamide, propane sulphonamide, and butanesulphonamide, to form the corresponding N-loweralkylsulfonylcarboxamide XXXVIII:

XXXIII 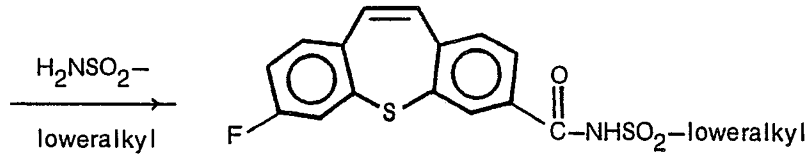


XXXVIII

(e) with 2-imino-3-methylthiazolidine to form the corresponding (3-methyl-2-thiazolidinylidene)carboxamide XXXIX:

XXXIII 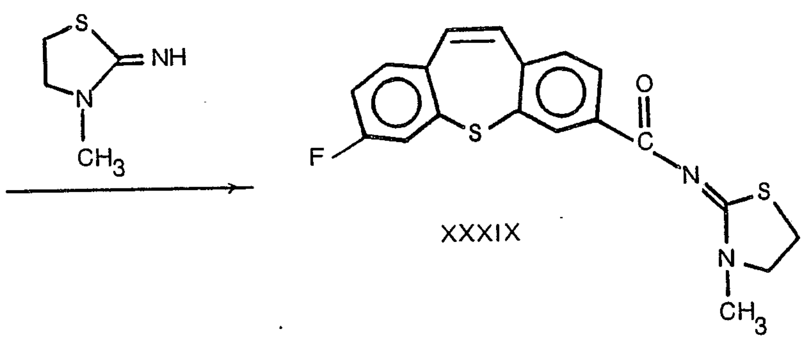

XXXIX

(f) with a lowrealkylanediol such as, for example, ethylene glycol, trimethylene glycol, and 1,4-butanediol, to form the corresponding hydroxyloweralkylester XL:

XXXIII 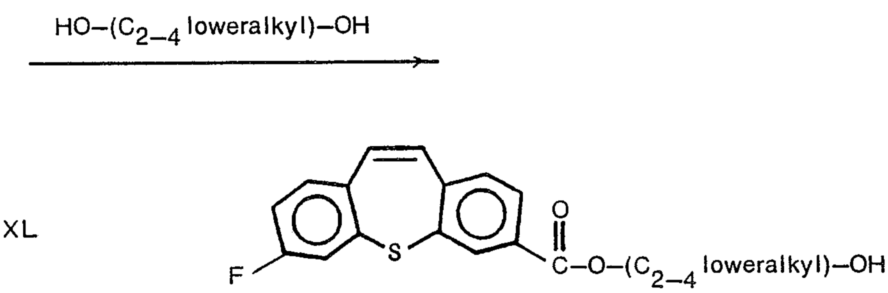


XL

(g) with an N,N-diloweralkylaminoloweralkanol such as, for example, N,N-dimethylethanolamine, N,N-diethylethanolamine, 3-(N,N-dimethylamino)propan-1-ol, and 4-(N,N-diethylamino)butan-1-ol, to form the corresponding N,N-diloweralkylaminoloweralkyl ester XLI:

XXXIII 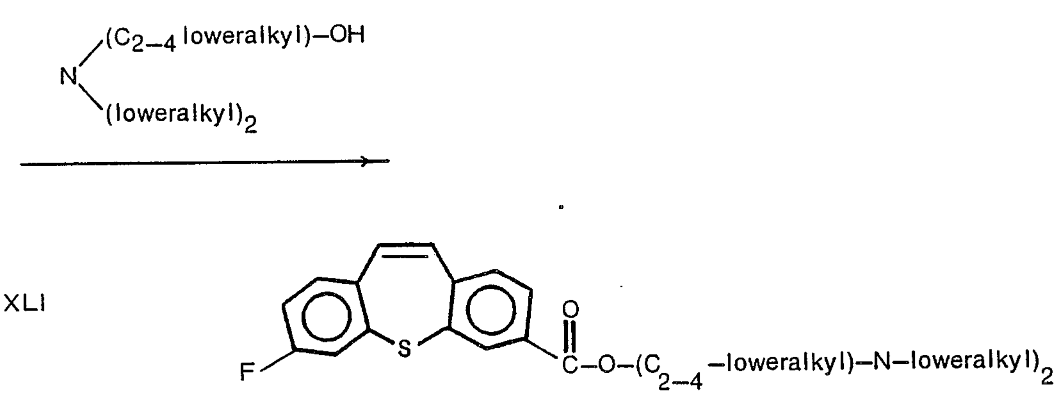

XLI

(h) with an amino acid such as, for example, glycine, alanine, and valine, to form the corresponding N-carboxyloweralkylcarboxamide XLII:

XXXIII

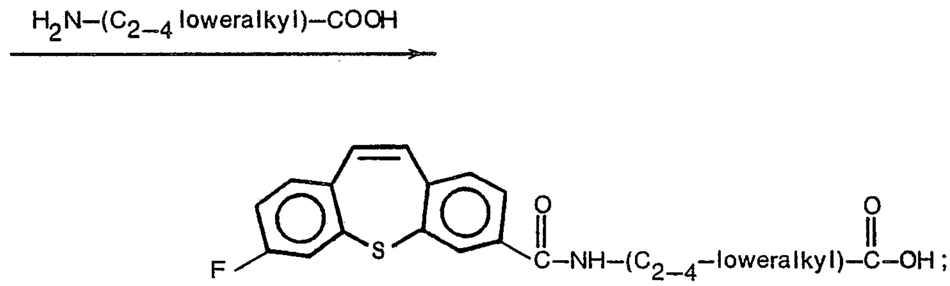


XLII

; and

(i) with an alkali metal salt of a hydroxyloweralkanoic acid such as, for example, hydroxyacetic acid, 3-hydroxybutyric acid, and β-hydroxypropionic acid, to form the corresponding carboxyloweralkyl ester XLIII:

XXXIII

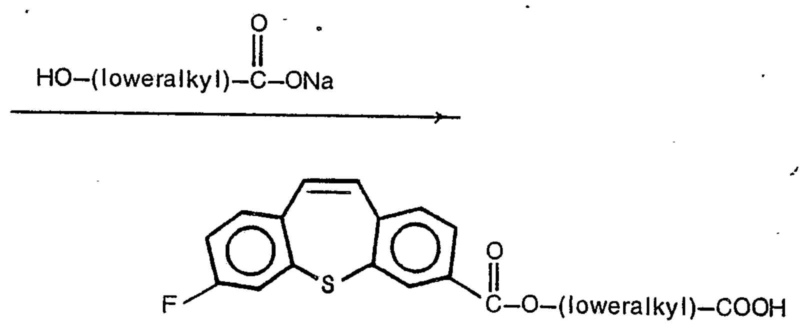


XLIII

Where the corresponding sulfinyl or sulfonyl derivatives are desired, the corresponding 5-oxide or 5,5-dioxide 3-carboxylic acid may be substituted for starting material XIX in the foregoing reaction sequence. Alternatively, it will be clear to those skilled in the art that the product esters and amides obtained in the foregoing reaction sequence may be oxidized by the techniques already described to obtain the corresponding sulfinyl or sulfonyl derivatives.

Those thiepins of this invention wherein the substituent at the 3-position is 3-hydroxy-1,2,5-thiadiazol-4-yl are prepared by refluxing a 3-cyano intermediate in formic acid in the presence of Raney nickel alloy for 1 to 2 hours in order to obtain the corresponding 7-fluoro-dibenzo[b,f]thiepin-3-carboxaldehyde. The aldehyde product is then converted into the corresponding 3-(2-aminoacetonitrile) by treatment with sodium cyanide in an alcoholic solvent saturated with ammonia and in the presence of ammonium chloride and ammonium hydroxide. The reaction usually is conducted at room temperature and requires from 8 to 16 hours for completion. The aminoacetonitrile so produced is treated with concentrated hydrochloric acid at room temperature for 20 to 45 minutes in order to obtain the corresponding 3-(2-aminoacetamide) which then is treated with sulfur monochloride in dimethylformamide to obtain the desired 7-fluoro-3-(3-hydroxy-1,2,5-thiadiazol-4-yl)-dibenzo[b,f]thiepin of formula XLIV. This reaction squence is illustrated in the following diagram.

XXVII

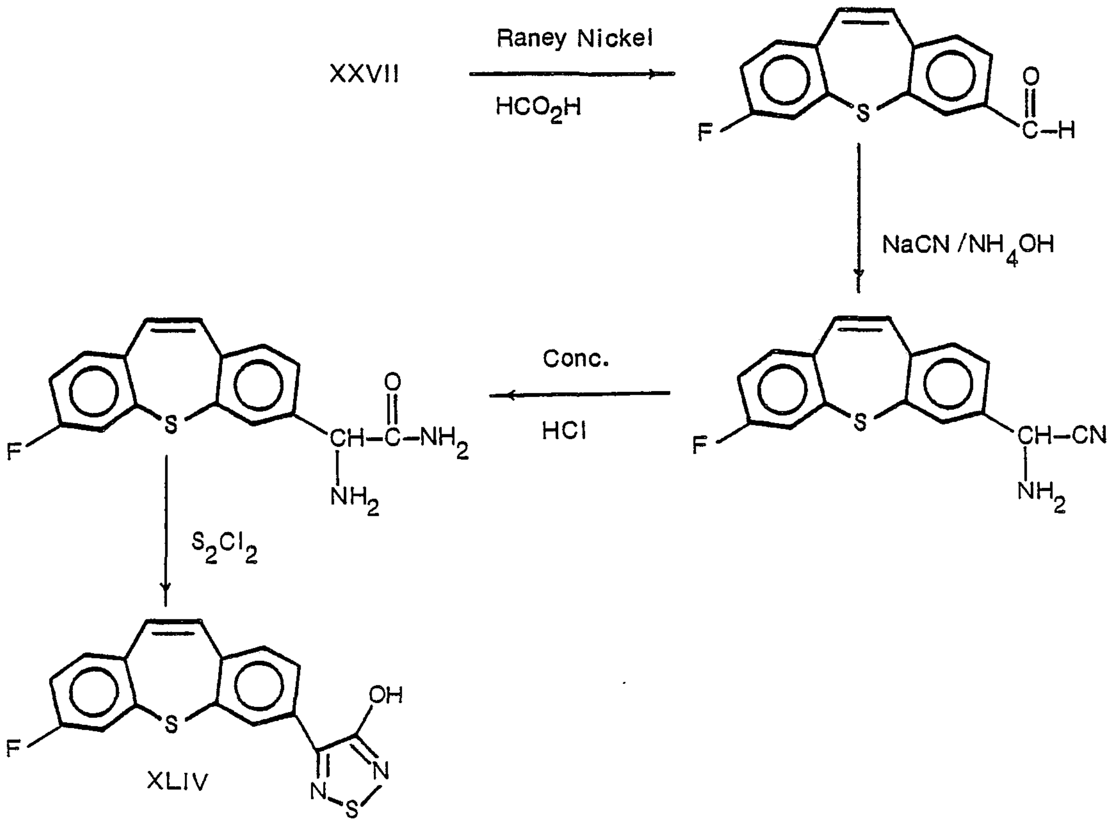


XLIV

The novel thiepins of this invention wherein the substituent at the 3-position is 4-hydroxy-2,5-dioxo-$\Delta^3$-pyrrolin-3-yl are prepared from the appropriately substituted 3-carboxylic acid by reducing the acid to the corresponding alcohol with borane in tetrahydrofuran. The reaction is conveniently carried out at room temperature under an inert atmosphere and usually requires 2 to 4 hours for completion. The alcohol then is brominated with phosphorus tribromide, and bromethyl compound so produced is treated with sodium cyanide to form the corresponding 3-cyanomethyl derivative. These reactions may be carried out at room temperature and usually require from 1 to 3 hours for completion. The cyanomethyl intermediate is then hydrolyzed to the corresponding acetic acid which is treated with thionyl chloride followed by ammonia to form the corresponding 3-acetamide derivative by techniques already described. The acetamide is then treated with diethyl oxalate in dimethylformamide in the presence of potassium *t*-butoxide to form the desired 3-(7-fluoro-dibenzo[b,f]thiepin-3-yl)-4-hydroxy-$\Delta^3$-pyrrolin-2,5-dione XLV. This reaction sequence is illustrated in the following diagram.

XIII

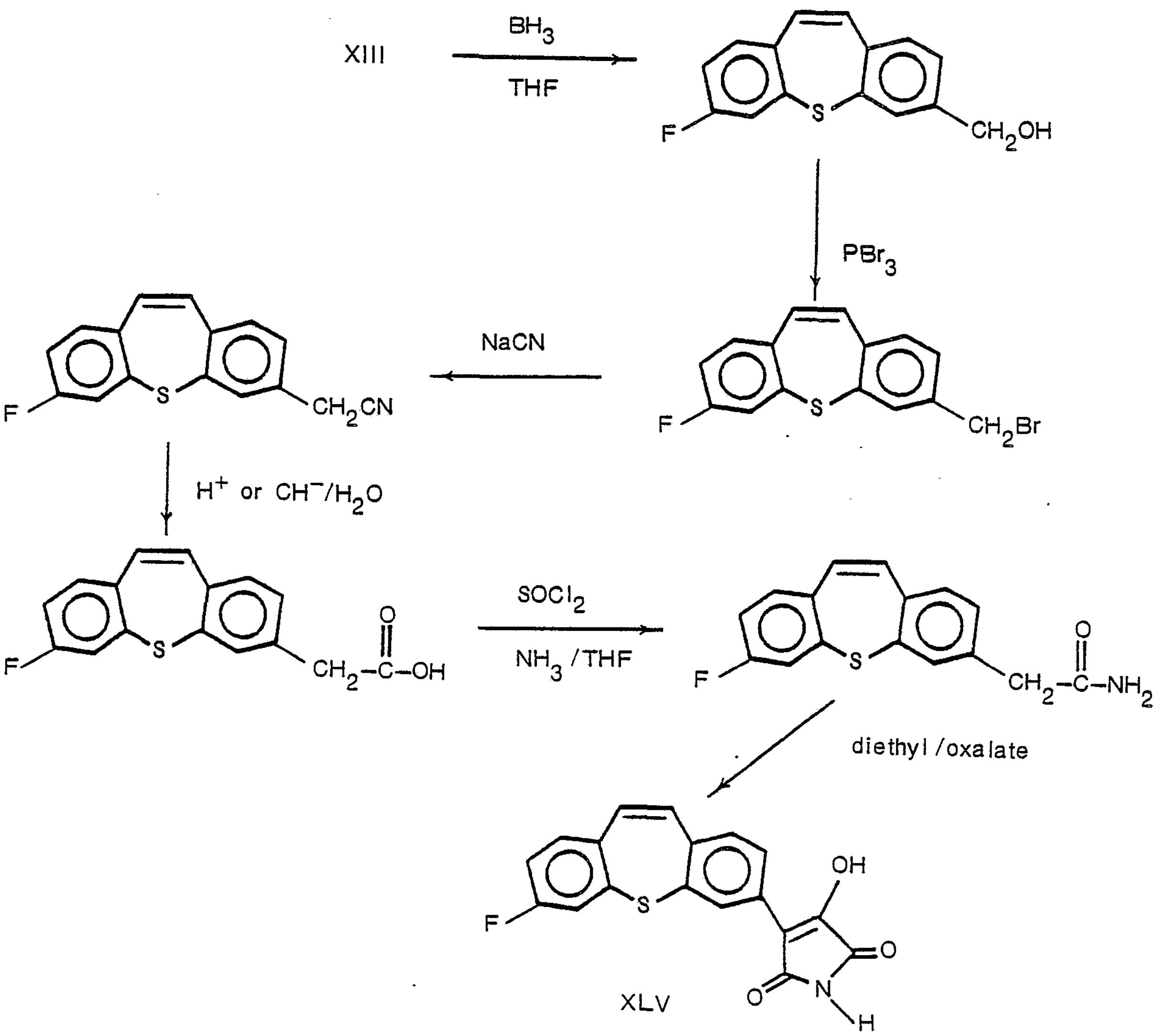


XLV

Where corresponding sulfinyl or sulfonyl derivatives are desired, the products of the four reaction schemes described immediately above may be oxidized by the techniques already described.

It will be noted that the reaction sequence described above affords not only thiepins of this invention wherein the substituent at the 3-position is 4-hydroxy-2,5-dioxo-$\Delta^3$-pyrrolin-3-yl, but in Steps A—D leads also to the preparation of those thiepins of this invention wherein the substituent at the 3-position is a loweralkanoic acid. Thus, Steps A—D, as described above, starting with the appropriately substituted 3-carboxylic acid, through reduction, bromination, cyanation, and hydrolysis, affords the corresponding 3-acetic acid derivative. Quite obviously, the described reduction, bromination, cyanation, and hydrolysis sequence can be repeated, employing the 3-acetic acid derivative as starting material, in order to obtain the corresponding propionic acid derivative which, in turn, can be employed as starting material for preparing the corresponding butyric acid derivative. In this manner, any desired 3-loweralkanoic acid derivative of the instant invention readily is prepared. Corresponding sulfinyl or sulfonyl derivatives are prepared by the oxidation techniques previously described.

The 3-cyanoloweralkyl intermediates obtained from Steps A—C in the reaction sequence described above also serve as intermediates in the preparation of other therapeutically active thiepins of formula I. Thus, for example, an appropriately substituted 3-cyanomethyl-7-fluoro-dibenzo[b,f]thiepin may be treated with sodium azide and ammonia by techniques previously described to form the corresponding 3-(1H-tetrazol-5-ylmethyl)-7-fluoro-dibenzo[b,f]thiepin and the product, if desired, can be oxidized to form the corresponding sulfinyl or sulfonyl derivative.

In accordance with the present invention a racemic mixture of a compound of formula IA or XII is resolved into its individual optical isomers. The optical isomerism is effected by the sulfoxide group which is tetrahedral in configuration and, therefore, an asymmetric center. It is surprising to discover that the resolution of the racemic mixture yields stable optically active isomers, one of which is unexpectedly potent in the activity possessed by the racemic mixture.

The highly active class of enantiomers has the absolute configuration shown in formula IIA relative to the sulfoxide substituent

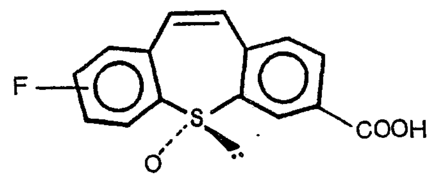

IIA

hereinafter referred to as S(−) 7-fluorodibenzo[b,f]thiepin-3-carboxylic acid-5-oxide.

The optically active dibenzo[b,f]thiepin derivatives of formula IIA hereinabove and the enantiomeric compounds of formula IIIA

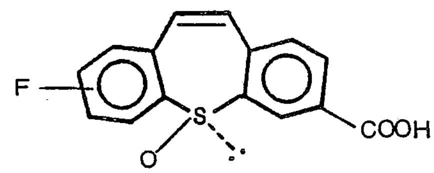

IIIA

hereinafter referred to as R(+) 7-fluorodibenzo[b,f]thiepin-3-carboxylic acid-5-oxide are prepared by the resolution of the racemic mixtures of isomers of formula IA hereinabove.

The preferred compositions of the invention are the substituted dibenzo[b,f]thiepin-3-carboxylic acids of the following structure

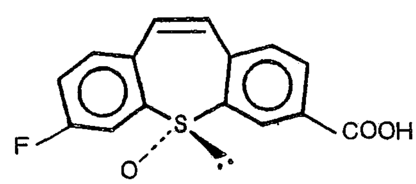

S(−) isomer of 7-fluorodibenzo[b,f]thiepin-3-carboxylic acids-5-oxide.

In accordance with one aspect of the present invention a diastereoisomeric mixture is formed by preparation of an amide derivative of 7-fluoro-dibenzo[b,f]thiepin-3-carboxylic acid-5-oxide and an optically active amine. Following formation of the diastereoisomeric compounds the two diastereoisomers are separated by chromatography and fractional crystallization. After separation and purification into individual components, each diastereoisomer is treated to regenerate the individual optical isomer which is then further purified by crystallization.

The diastereoisomeric derivative 7-fluorodibenzo[b,f]thiepin-5-oxide may be prepared in one of two ways. In one method, an optical derivative of the corresponding thiepin is prepared followed by oxidation to the corresponding diastereoisomeric 5-oxide. In an alternative method the racemic 7-fluorodibenzo[b,f]thiepin-3-carboxylic acid-5-oxide is reacted with an optically active amine or alcohol to form the corresponding diastereoisomeric salt, amide or ester.

In one method of resolution, the racemic 7-fluorodibenzo[b,f]thiepin-3-carboxylic acid is converted to an amide of an optically active amine by first converting said acid to the corresponding acid halide followed by reaction of the acid halide with an optically active amine to produce a 7-fluorodibenzo[b,f]thiepin-3-carboxylic acid optically active amide enantiomer. The enantiomer is then oxidized to the corresponding 5-oxide, thus creating a new center of assymetry at the 5-position and producing a pair of diastereoisomers, which are separated by chromatography and/or crystallization into the individual diastereoisomers.

In the alternative method of preparing the mixture of diastereoisomers the formation of the amide is carried out by using the 5-oxide of the acids as starting material. Thus the 7-fluorodibenzo[b,f]thiepin-3-carboxylic acid-5-oxide is converted in the manner described for the corresponding thiepin compound first to the acid chloride and then reacted with an optically active amide to produce a mixture of diastereoisomeric amides of the (−) acid and the (+) acid.

In a preferred embodiment of the process employing resolution of the optically active amide the racemic 7-fluorodibenzo[b,f]thiepin-3-carboxylic acid is converted to an acid chloride followed by reaction with S(−) α-methylbenzylamine to form 7-fluorodibenzo[b,f]thiepin-3 (N-α-methylbenzylcar-

boxamide). This optically active amide is then converted to a mixture of diastereoisomers by oxidation to the corresponding sulfoxide 7-fluorodibenzo[b,f]thiepin 3(N-α-methylbenzylcarboxamide)-5-oxide, thus forming a new center of assymetry at the 5 position. This mixture of diastereoisomers is separated into its component parts by chromatography followed by crystallization of the individual diastereoisomers.

The chromatography is preferably carried out using silica gel suspended in a mixture of ethyl acetate and chloroform. The eluting agent used is a mixture of ethyl acetate and chloroform. The diastereoisomers are eluted in two readily separable fractions based on polarity. The least polar fraction which is eluted primarily in the early stages of elution is identified as

Diastereoisomer A
7-fluorodibenzo[b,f]thiepin-3(N-α-methylbenzyl)-carboxamide-5-oxide of the following absolute configuration

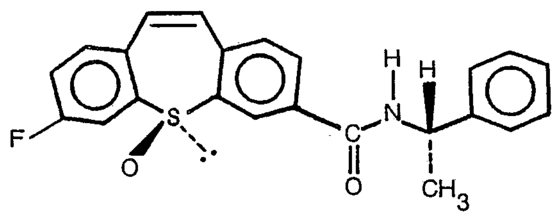

Diastereoisomer B
7-fluorodibenzo[b,f]thiepin-3(N-α-methylbenzyl)-carboxamide-5-oxide of the following absolute configuration

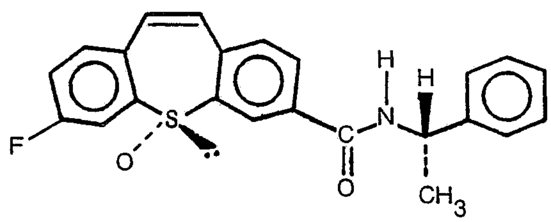

The absolute configuration depicted in the structural formulas shown hereinabove and elsewhere in the subject application are determined by single crystal X-ray analysis of the α-methylbenzylamides and by reference to such amides using synthetic conversions which do not interfere with the center of asymmetry.

The individual diastereoisomers are converted to the free acids by hydrolysis preferably in the presence of aqueous acid. In this manner Diastereoisomer A is converted to

(+)7-fluorodibenzo[b,f]thiepin-3-carboxylic acid-5-oxide of the formula

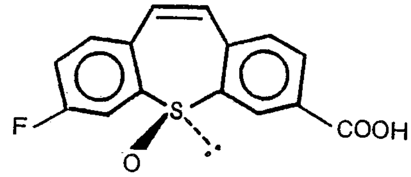

This (+)7-fluorodibenzo[b,f]thiepin-3-carboxylic acid-5-oxide compound has no measurable biological activity as prostaglandin antagonists but are useful as intermediates in the preparation of the active isomer B. Thus such compounds may be converted to the racemic form by reduction to the sulfide followed by reoxidation or by direct treatment with trifluoro acetic anhydride.

In similar manner diastereoisomer B is hydrolyzed, preferably in aqueous acid, to

S(−) 7-fluorodibenzo[b,f]thiepin-3-carboxylic acid-5-oxide of the following formula

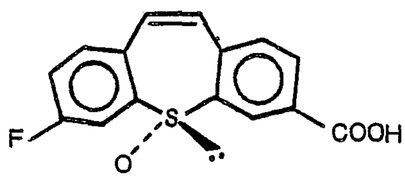

Surprisingly, the above 7-fluoro acid of negative optical rotation has at least twice the potency of the racemic form in antagonizing the contractile action caused by certain prostaglandins or prostaglandin like substance and thus are particularly useful in the treatment of disease conditions.

As noted above, pharmaceutically acceptable salts of the novel thiepins are also included within the scope of this invention. The term "pharmaceutically acceptable salts" is intended to include salts derived from pharmaceutically acceptable, non-toxic acids and bases such as, for example, ammonium salts; alkali metal salts such as sodium and potassium salts; alkaline-earth metal salts such as magnesium and calcium salts; salts of organic bases such as amine salts derived from (mono, di and tri)-(loweralkyl or loweralkanol)amines such as trimethylamine, dimethylamine, and triethanolamine; salts derived from heterocyclic amines such as piperidine, pyridine, piperazine, and morpholine; and salts derived from pharmaceutically acceptable acids such as hydrochloric acid, sulfuric acid, tartaric acid, and propionic acid.

The following Examples illustrate various processes in accordance with the present invention. The abbreviation "mmole" means "millimole".

Example 1

Preparation of 7-Fluoro-dibenzo[b,f]thiepin-3-carboxylic Acid-5-oxide

*Step A: 2-(3-Aminophenylthio)-4-carboxyphenylacetic Acid*

Dissolve 9.9 g. potassium hydroxide in 75 ml. water and add 10.2 g. (33.3 mmoles) of 2-iodo-4-carboxyphenylacetic acid, followed by 6.26 g. (50 mmoles) 3-aminothiophenol and 2.54 g. (40 mmoles) of copper powder. Heat the resulting mixture under a nitrogen atmosphere at reflux temperature for a period of 4 hours. Filter the mixture while hot and crystallize the product from the filtrate by cooling and acidification with 10% aqueous hydrochloric acid while stirring. Filter the product and triturate with ether to remove non-acidic by-products. Stir the crude product thus obtained in aqueous sodium bicarbonate for about 2 hours and filter the mixture. The product is isolated from the filtrate by treatment with aqueous hydrochloric acid to precipitate the acetic acid intermediate. The product is isolated by filtration, water-washing, and air-drying.

*Step B: 7-Amino-10,11-dihydro-10-oxo-dibenzo[b,f]thiepin-3-carboxylic Acid*

Add 6.6 g. of the product of the preceding reaction step to 85 g. commercial polyphosphoric acid and heat to 95—100°C. with stirring for a period of 3 hours. Following the reaction, cool the mixture and add carefully 1000 cc of ice-cold water. Filter the crude product which precipitates and repeat the treatment with polyphosphoric acid for a period of about 1 hour. The product is again precipitated by the addition of water and recovered by filtration.

*Step C: 7-Amino-10-hydroxy-10,11-dihydro-dibenzo[b,f]thiepin-3-carboxylic Acid*

Suspend the crude product from the preceding step in water and add 12 g. of sodium bicarbonate with stirring. When carbon dioxide evolution ceases, add 3 g. sodium borohydride to the suspension in small portions, during which time all salts dissolve to form a brown solution. Allow this solution to stand at room temperature for about 18 hours.

Acidify the entire reaction mixture with 10% aqueous hydrochloric acid to a pH of 5. During the acidification, add ethyl acetate as needed to control the frothing. Stir the mixture and filter the resulting two-phase system to remove insoluble materials. Partition the filtrate between water and ethyl acetate. (The product is extracted preferentially into the ethyl acetate layer.) The product is obtained by evaporation of the ethyl acetate extract under reduced pressure and drying the resulting residual product.

*Step D: 7-Amino-dibenzo[b,f]thiepin-3-carboxylic acid methyl ester*

Dissolve 100 mg. of crude hydroxy acid from the preceding step in 2 cc. acetic acid and heat the solution on a steam bath. Add 0.5 cc. sulfuric acid, heat, and stir for approximately 5 minutes. The reaction mixture is then cooled and diluted with water, resulting in the precipitation of solid product, 7-amino-dibenzo[b,f]thiepin-3-carboxylic acid.

Heat the product carboxylic acid in 30 cc. methanol containing 1 cc. of sulfuric acid overnight to effect esterification and recover the title product. Recovery of the product is accomplished by evaporation of the solvent nearly to dryness, leaving a residual crude ester. Dissolve the residue in chloroform and wash with water. Evaporate the chloroform extract, leaving a solid residue as a product. Chromatograph the product by use of a column of 500 g. silica gel. Introduce the crude product to the top of the column, and elute with 10% ethyl acetate in toluene. Recover the desired methyl ester from the eluate by evaporation of the

solvent, followed by trituration of the residual material in hexane. The resulting product is a yellow solid having a m.p. of 139—145°C.

*Step E: 7-Fluoro-dibenzo[b,f]thiepin-3-carboxylic Acid Methyl Ester*

Add 1.54 g. of the methyl ester of 7-amino-dibenzo[b,f]thiepin-3-carboxylic acid to a stirred solution of 5.5 cc. of water and 5.5 cc. of 40—50% fluoboric acid, producing a gummy-like solid. Cool the mixture in an ice bath and add a solution of 385 mg. sodium nitrite (5.6 mmoles) in a dropwise manner, during which time the gummy solid material turns into a yellow solid. Stir the entire reaction mixture at ice-bath temperature for 2 hours, then filter and wash the resulting product with 25% fluoboric acid solution. After air-drying, repeat the procedure of treating the solid with 11 cc. of water and 11 cc. of 40—50% fluoboric acid at ice-bath temperature. Isolate the crude product in the same manner as in the previous procedure by filtration and air-drying on the filter. The solid material is pyrolyzed in 100 cc. xylene by stirring at 140°C. for 2½ hours. The crude product obtained by evaporation of the xylene solution is chromatographed on a column of silica gel and eluted with toluene. Removal of toluene by evaporation produces the product as a yellow oil which crystallizes on standing; m.p. 132—135°C.

Hydrolysis of 1.0 g of the product ester by treatment with 20 cc. of 10% aqueous sodium hydroxide and 20 cc. ethanol at 25°C. for two hours produces 7-fluorodibenzo[b,f]thiepin-3-carboxylic acid which is precipitated with water after acidification of the reaction mixture, m.p. 280—82°C.

*Step F: 7-Fluoro-dibenzo[b,f]thiepin-3-carboxylic acid-5-oxide methyl ester*

Dissolve the product from the previous step in 20 cc. methylene chloride and cool the solution in an ice bath. Add 268 mg. 85% m-chloroperbenzoic acid (90% of a molar equivalent), and stir the resulting mixture at ice-bath temperature for about 1 hour. Add 1 g. calcium hydroxide to the reaction mixture and stir for 5 minutes. Dilute the reaction mixture with methylene chloride and filter through celite. The resulting filtrate containing the product is evaporated to produce as a residue solid sulfoxide product. Chromatograph the product on a column of silica gel and elute the product with methylene chloride. The product is rechromatographed on a column of silica gel and eluted with a mixture of 20% ethyl acetate in toluene. The product is obtained by evaporation of the eluate to produce solid crystalline material; m.p. 178—181°C.

*Step G: 7-Fluoro-dibenzo[b,f]thiepin-3-carboxylic acid-5-oxide*

Stir 290 mg. of the ester from the preceding step in a mixture of 12 cc. dioxane and 12 cc. 10% aqueous sodium hydroxide for about 2 hours during which time the sodium salt precipitates from solution. Dilute the reaction mixture with water to dissolve the salt, and precipitate the acid by acidification with 10% hydrochloric acid. The product is recovered by filtration and air-dried; m.p. 255—257°C. with decomposition.

Example 2

Preparation of 7-fluorodibenzo[b,f]thiepin-3(N-α-methylbenzyl)carboxamide

9.52 g. of 7-fluorodibenzo[b,f]thiepin-3-carboxylic acid (35 mmoles) is dissolved in 350 cc. tetrahydrofuran, and 4.24 g. triethylamine (42 mmoles) is added. The solution is cooled in an ice-water bath and a solution of 4.18 g. ethyl chloroformate (38.5 mmoles) in 10 cc. tetrahydrofuran is added dropwise. The suspension is stirred in the cold for 15 minutes then a solution of 8.49 g. S-(−)-α-methylbenzylamine (70 mmoles) in 15 cc. tetrahydrofuran is added dropwise. The resulting mixture is stirred in the cold for one hour, then filtered, and the filtrate stripped down. The residue is dissolved in chloroform, concentrated, and injected on a column of silica gel; elution is done with 5 percent ethylacetate/toluene. The product is recovered as a yellow oil and is used directly in the following example.

Example 3

Preparation of 7-fluorodibenzo[b,f]thiepin-3(N-α-methylbenzylcarboxamide)-5-oxide as a mixture of R,S and S,S-diastereoisomers

7.05 g. of 7-fluorodibenzo[b,f]thiepin-3)N-α-methylbenzyl)carboxamide S-isomer is dissolved in 350 cc. methylene chloride, and the solution cooled in an ice-and-water bath. 3.65 g. 85 percent m-chloroperbenzoic acid (95 percent of 19 mmoles) is added; and the mixture stirred in the cold for one hour, then diluted with 125 cc. methylene chloride and 6 g. calcium hydroxide are added. The mixture is stirred for 5 minutes, then filtered through celite. The filtrate is stripped to an oil which is chromatographed on 400 g. silica gel to yield the title compound.

Example 4

Separation of diastereoisomers of 7-fluorodibenzo[b,f]thiepin-3-carboxylic acid-5-oxide

The mixture of diastereoisomers is separated by extensive high performance liquid chromatography (HPLC) using a pre-packed column of silica gel and eluting with 10% ethylacetate in toluene. The least polar compound is the R,S-diastereoisomer, while the more polar material is the S,S-diastereoisomer.

Fractions containing the least polar R,S-diastereoisomer in ≥80—85% purity were combined and crystallized from toluene, affording crystals >99% of the R,S-diastereoisomer. m.p.: 186°—188°C.

Fractions containing the more polar S,S-diastereoisomer in ≥80—85% purity were combined and crystallized from toluene, affording crystals of ≥99% pure S,S-diastereoisomer, m.p.: 195°—196°C.

The absolute configuration of the S,S-diastereoisomer was established by single crystal X-ray analysis.

Example 5

Hydrolysis of S,S-7-fluorodibenzo[b,f]thiepin-3(N-α-methylbenzyl)carboxamide-5-oxide to S(−)-7-fluorodibenzo[b,f]thiepin-3-carboxylic acid-5-oxide

4.15 g. S,S-7-fluorodibenzoWb,fwthiepin-3(N-α-methylbenzyl)carboxamide-5-oxide is heated in a mixture of 100 cc. glacial acetic acid and 100 cc. 50% sulfuric acid at 100°—105°C for a period of 23 hours. The reaction mixture is diluted with 300 cc. water and the solid product is recovered by filtration, air dried, slurried in hexane and refiltered to obtain a fluffy cream-colored solid m.p. 257°C. dec. resolid m.p. 315°—320°C.

[α] = −28.77°C 1% solution in 2:1 5% of $NaHCO_3$/ethanol.

Example 6

Hydrolysis of R,S 7-fluorodibenzo[b,f]thiepin-3-(N-α-methylbenzyl)carboxamide-5-oxide to R(+)-7-fluorodibenzo[b,f]thiepin-3-carboxylic acid-5-oxide

An equivalent amount of isomer A is treated with acetic and sulfuric acid in the manner described in Example 5 with production of the desired product $[\alpha]_D$: +26.80 1% solution in 2:1 5% aqueous $NaHCO_3$/ ethanol m.p.: 256°C dec, resolidifies, m.p. 314°—318°C.

Example 7

7-Fluorodibenzo[b,f]thiepin-3-carboxylic acid

The title compound is produced starting with 3-fluorothiophenol and employing the process outlined in the flow sheet on page 4 of the specification and described in detail on page 4 of the specification. Intermediates in this process are

2-(3-fluorophenythio)-4-carboxy-phenyl acetic acid,

10-oxo-10,11-dihydro-dibenzothiepin;

10-hydroxy-10,11-dihydrodibenzothiepin

The 10 hydroxy compound is dehydrated by treatment with a catalytic amount of a strong acid to produce the title compound M.P. 280—82°.

The compounds of formula I and IA are useful in the treatment or prophylaxis of mammalian disease conditions where excessive undesirable contractile activity of prostaglandins, such as $PGF_{2\alpha}$, or prostaglandin biosynthetic intermediates contributes. These conditions include asthma, inflammatory states such as arthritis, allergy, diarrhea, hypertension, angina, platelet aggregation, cerebral spasm, premature abortion and dismenorrhea. In particular, they are of value in reaginic mediated asthma (extrinsic asthma).

The magnitude of a prophylactic or therapeutic dose of compound of formula I will, of course, vary with the nature and the severity of the condition to be treated and with the particular compound of formula I and its route of administration. In general, the dose range lies within the range of 0.2 mg. to 100 mg. per kg. body weight of a mammal.

The pharmaceutical compositions of the present invention comprise a compound of formula I as an active ingredient, and may also contain pharmaceutically acceptable carrier and optionally other therapeutic ingredients. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the condition being treated and on the nature of the active ingredient. They may be conveniently presented in unit dosage form and prepared by any of the methods well known in the art of pharmacy.

For use where a composition for intravenous administration is employed, a suitable dosage range is from 0.2 to 10 mg. (preferably 1 to 5 mg.) of a compound of formula I per kg. of body weight per day and in the case where an oral composition is employed a suitable dosage range is about, e.g. 1 to 50 mg. of a compound of formula I per kg. of body weight per day, preferably from 10 to 40 mg./kg.

Pharmaceutical compositions of the present invention suitable for oral administration and by inhalation in the case of asthma therapy may be presented as discrete units such as capsules, cachets or tablets each containing a predetermined amount of the active ingredient; as a powder or granules; or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Moulded tablets may be made by moulding in a suitable machine, a mixture of the powdered compound moistened with an inert liquid diluent. Desirably, each tablet contains from 50 mg. to 500 mg. of the active ingredient and each cachet or capsule contains from 50 mg. to 500 mg. of the active ingredient.

Although the invention has been described in the foregoing specification in terms of the use of the novel thiepin disclosed herein in the treatment and control of human and warmblooded animal disease conditions characterized by excessive undesirable contractile activity of prostaglandins and prostaglandin biosynthetic intermediates, and particularly of asthma, it will be recognized by those skilled in the art that, in addition to the involvement of contractile prostaglandins in chronic obstructive lung disease (e.g., asthma), prostaglandins play a role in other allergic conditions as well as in inflammation, diarrhea, hypertension, angina, cerebral spasm, premature abortion and dismenorrhea. Also, the thiepins of this invention are potent $TXA_2$ biosynthesis inhibitors, inhibiting platelate aggregation, and can be useful in diseases such as atherosclerosis, variant anginal and myocardial infarction. Applicants consider application of the thiepins disclosed and claimed herein to the treatment and control of such disease conditions to be obvious equivalents to the invention as disclosed by applicants and to fall within the scope of the invention.

**Claims**

1. A compound of the formula:

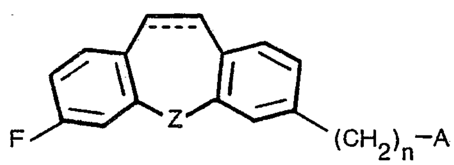

in which n is 0, 1, 2, 3 or 4; Z is thio, sulfinyl, or sulfonyl; A is 5-tetrazolyl, 3-hydroxy-1,2,5-thiadiazol-4-yl, 4-hydroxy-2,5-dioxo-$\Delta^3$-pyrrolin-3-yl, or —CO—$R_2$, where $R_2$ is hydroxy, $C_{1-4}$ alkyl, $C_{1-4}$ alkoxy, N,N-di($C_{1-4}$ alkyl)-amino-($C_{1-4}$ alkoxy), $C_{1-4}$ hydroxyalkoxy, carboxy-($C_{1-4}$ alkoxy), amino, N-($C_{1-4}$ alkyl)amino, N,N-di($C_{1-4}$ alkyl)amino, $C_{1-4}$ alkylsulfonylamino, carboxy-($C_{1-4}$ alkyl)amino, carboxamido-($C_{1-4}$ alkyl)-amino, or 2-imino-3-methylthiazolidinyl; and the dotted line indicates either an olefinic bond or saturation at the 10,11-position; and compounds that are pharmaceutically acceptable salts thereof.

2. 7-Fluorodibenzo[b,f]thiepin-3-carboxylic acid-5-oxide.

3. S-(−) 7-fluorodibenzo[b,f]thiepin-3-carboxylic acid-5-oxide of the formula:

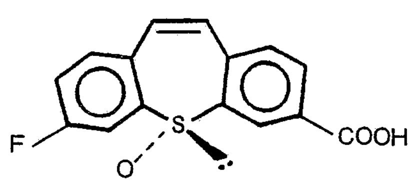

4. R(+) 7-fluorodibenzo[b,f]thiepin-3-carboxylic acid-5-oxide of the formula:

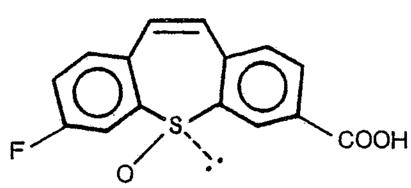

5. A composition for treating undesirable contractile activity of prostaglandins consisting essentially of a pharmaceutically acceptable carrier and a therapeutically effective amount of a compound as claimed in Claim 1, 2, 3 or 4.

6. A process for preparing a compound as claimed in Claim 1 but in which A is 5-tetrazolyl or carboxy that comprises hydrolysing a compound of the formula:

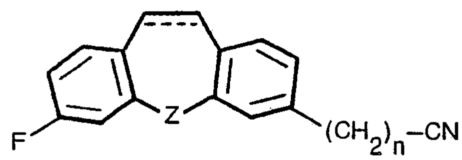

with an acid or base to form the carboxyl compound or reacting the compound of the said formula with an azide ion to form the 5-tetrazolyl compound.

7. A method of resolving the racemic mixture 7-fluorodibenzo[b,f]thiepin-3-carboxylic acid-5-oxide into its enantiomeric forms, that comprises forming a diastereoisomeric mixture of (−) 7-fluorodibenzo[b,f]thiepin-3-carboxylic acid-5-oxide optically active derivative and (+) 7-fluorodibenzo[b,f-]thiepin-3-carboxylic acid-5-oxide optically active derivative, separating the said mixture into the individual diastereomers by crystallization or chromatography and converting the individual diastereoisomers into (+) 7-fluorodibenzo[b,f]-3-carboxylic acid-5-oxide and (−) fluorodibenzo[b,f-]thiepin-3-carboxylic acid-5-oxide.

8. A process for the preparation of a compound of the formula:

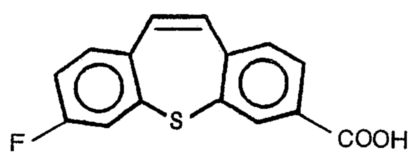

that comprises dehydrating a compound of the formula:

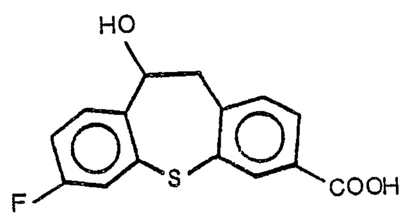

**Patentansprüche**

1. Eine Verbindung der Formel:

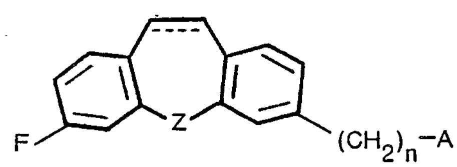

worin n 0, 1, 2, 3 oder 4 ist; Z Thio, Sulfinyl oder Sulfonyl ist; A 5-Tetrazolyl, 3-Hydroxy-1,2,5-thiadiazol-4-yl, 4-Hydroxy-2,5-dioxo-$\Delta^3$-pyrrolin-3-yl oder —CO—$R_2$ ist, worin $R_2$ Hydroxy, $C_{1-4}$-Alkyl, $C_{1-4}$-Alkoxy, N,N-Di($C_{1-4}$-alkyl)-amino-($C_{1-4}$-alkoxy), $C_{1-4}$-Hydroxyalkoxy, Carboxy-($C_{1-4}$-alkoxy), Amino, N-($C_{1-4}$-Alkyl)-amino, N,N-Di($C_{1-4}$-alkyl)amino, $C_{1-4}$-Alkylsulfonylamino, Carboxy-($C_{1-4}$-alkyl)amino, Carboxamido-($C_{1-4}$-alkyl)amino oder 2-Imino-3-methylthiazolidinyl ist; und die strichlierte Linie entweder eine olefinische Bindung oder eine Sättigung in der 10,11-Stellung anzeigt; und Verbindungen, die pharmazeutisch annehmbare Salze davon sind.

2. 7-Fluordibenzo[b,f]thiepin-3-carbonsäure-5-oxid.

3. S-(−)-7-Fluordibenzo[b,f]thiepin-3-carbonsäure-5-oxid der Formel:

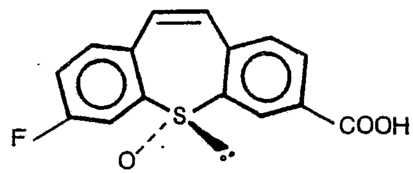

4. R(+)-7-Fluordibenzo[b,f]thiepin-3-carbonsäure-5-oxid der Formel:

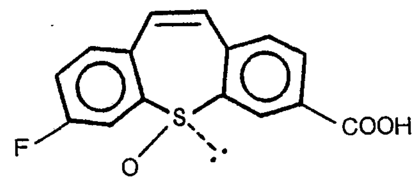

5. Eine Zusammensetzung zur Behandlung unerwünschter kontraktiler Aktivität von Prostaglandinen, bestehend im wesentlichen aus einem pharmazeutisch annehmbaren Träger und einer therapeutisch wirksamen Menge einer Verbindung, wie in Anspruch 1, 2, 3 oder 4 beansprucht.

6. Ein Verfahren zur Herstellung einer Verbindung, wie in Anspruch 1 beansprucht, worin aber A 5-Tetrazolyl oder Carboxy ist, umfassend das Hydrolysieren einer Verbindung der Formel:

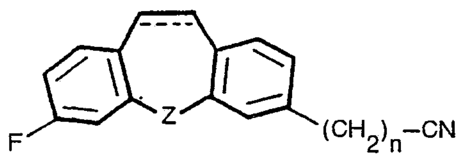

mit einer Säure oder Base unter Bildung der Carboxylverbindung oder die Umsetzung der Verbindung der genannten Formel mit einem Azidion unter Bildung der 5-Tetrazolylverbindung.

7. Ein Verfahren zur Trennung des racemischen Gemisches von 7-Fluordibenzo[b,f]thiepin-3-carbonsäure-5-oxid in seine enantiomeren Formen, welches die Bildung eines diastereoisomeren Gemisches vom optisch aktiven Derivat des (−)-7-Fluordibenzo[b,f]thiepin-3-carbonsäure-5-oxids und vom optisch aktiven Derivat des (+)-7-Fluordibenzo[b,f]thiepin-3-carbonsäure-5-oxids, die Trennung dieses Gemisches in die einzelnen Diastereomeren durch Kristallisation oder Chromatographie und die Umwandlung der einzelnen Diastereoisomeren in (+)-7-Fluordibenzo[b,f]thiepin-3-carbonsäure-5-oxid und (−)-7-Fluordibenzo[b,f]thiepin-3-carbonsäure-5-oxid umfaßt.

8. Ein Verfahren zur Herstellung einer Verbindung der Formel:

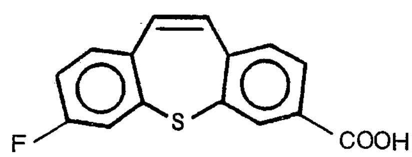

welches die Dehydratisierung einer Verbindung der Formel:

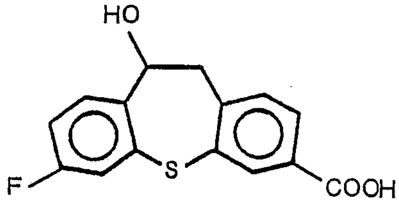

umfaßt.

**Revendications**

1. Un composé de formule:

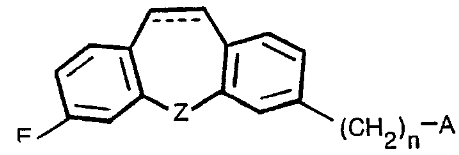

dans laquelle n est 0, 1, 2 ou 3; Z est un thio, un sulfinyle ou un sulfonyle; A est un 5-tétrazolyle, un 3-hydroxy-1,2,5-thiadiazole-4-yle, un 4-hydroxy-2,5-dioxo-$\Delta^3$-pyrroline-3-yle ou —CO—$3_2$ où $R_2$ est un hydroxy, un alkyle en $C_{1-4}$, un alcoxy en $C_{1-4}$, un N,N-di(alkyl en $C_{1-4}$)amino-(alcoxy en $C_{1-4}$), un hydroxyalcoxy en $C_{1-4}$, un carboxy-(alcoxy en $C_{1-4}$), un amino, un N-(alkyl en $C_{1-4}$)amino, un N,N-di(alkyl en $C_{1-4}$)amino, un alkylsulfonylamino en $C_{1-4}$; un carboxy-(alkyl en $C_{1-4}$)amino, un carboxamido-(alkyl en $C_{1-4}$)-amino ou un 2-imino-3-méthylthiadiazolinyle; et le pointillé indiqué soit une liaison oléfinique, soit une saturation de la position 10,11; et les composés qui sont leurs sels acceptables en pharmacie.

2. Acide 7-fluorodibenzo(b,f)thiépine-3-carboxylique-5-oxyde.

3. Acide S-(−) 7-fluorodibenzo(b,f)thiépine-3-carboxylique-5-oxyde de formule:

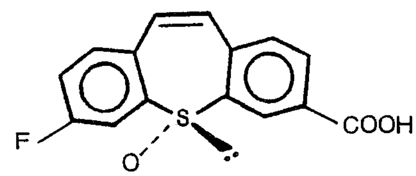

4. Acide R(+) 7-fluorodibenzo(b,f)thiépine-3-carboxylique-5-oxyde de formule:

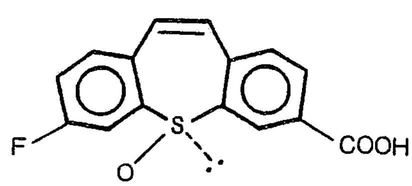

5. Une composition pour le traitement de l'activité constrictive indésirable des prostaglandines constituée essentiellement d'un support acceptable en pharmacie et d'une quantité thérapeutique efficace d'un composé comme revendiqué dans la revendication 1, 2, 3 ou 4.

6. Un procédé pour préparer un composé comme revendiqué dans la revendication 1, mais dans lequel A est un 5-tétra-zolyle ou un carboxy, qui comprend l'hydrolyse d'un composé de formule:

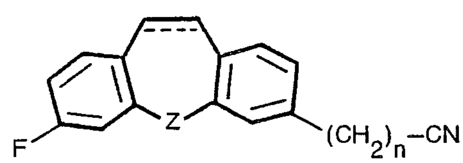

avec un acide ou une base pour former le composé carboxylique, ou la réaction du composé ayant ladite formule avec un ion azide pour former le composé 5-tétrazolylique.

7. Un procédé de dédoublement du mélange racémique de l'acide 7-fluorodibenzo(b,f)thiépine-3-carboxylique-5-oxyde en ses formes énantiomères, qui comprend la formation d'un mélange diastéréo-isomère de dérivés optiquement actifs de l'acide (−) 7-fluorodibenzo(b,f)thiépine-3-carboxylique-5-oxyde et de dérivés optiquement actifs de l'acide (+) 7-fluorodibenzo(b,f)thiépine-3-carboxylique-5-oxyde, la

séparation dudit mélange en les diastéréo-isomères individuels par cristallisation ou par chromatographie et la conversion des diastéréo-isomères individuels en l'acide (+) 7-fluorodibenzo(b,f)-3-carboxylique-5-oxyde et l'acide (−) 7-fluorodibenzo(b,f)thiépine-3-carboxylique-5-oxyde.

8. Un procédé pour la préparation d'un composé de formule:

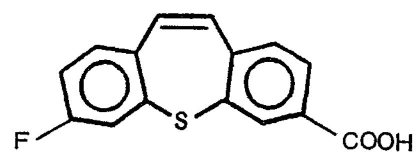

qui comprend la déshydratation d'un composé de formule: